# EUROPEAN PATENT SPECIFICATION

(11) **EP 2 385 850 B1**
(45) Date of publication and mention of the grant of the patent: **26.03.2014**
(21) Application number: 09795646.0
(22) Date of filing: 18.12.2009
(51) Int. Cl.: A61M 1/34, A61M 1/36, A61M 1/38

(54) **System for obtaining immunoglobulin from blood**
System zur Gewinnung von Immunglobulin aus Blut
Système pour obtenir une immunoglobuline à partir de sang

(30) Priority: 19.12.2008 US 339900
(43) Date of publication of application: 16.11.2011
(73) Proprietor: Baxter International Inc., Deerfield, IL 60015 (US); Baxter Healthcare S.A., 8152 Glattpark (Opfikon) (CH)
(72) Inventor: MIN, Kyungyoon, Kildeer IL 60047 (US); BACKES, Larry, Libertyville IL 60048 (US); RAUSA, Francisco, M., III, Vernon Hills IL 60061 (US); BAIRSTOW, Shawn, F., Gurnee IL 60031 (US); RAMACHANDRAN, Sindhu, Lake Zurich IL 60047 (US); POKROPINSKI, Sharon, Schaumburg IL 60193 (US)
(74) Representative: Potter Clarkson LLP
(86) International application number: PCT/US2009/068753
(87) International publication number: WO 2010/071809

(56) References cited:
- WO-A1-95/31209
- WO-A2-02/094344
- US-A- 4 705 628
- US-A- 5 782 792
- US-A- 5 817 528
- US-A1- 2002 009 445

## Description

### FIELD

The present disclosure relates generally to systems and methods for obtaining and/or isolating immunoglobulin (*e.g.,* IgG). Isolated immunoglobulin from a donor may be conveyed (*e*.*g*., directly infused) to a recipient. Also disclosed are methods for treating diseases or disorders, including those associated with immunodeficiency, by administering immunoglobulin isolated from a donor by the systems of the present disclosure to a recipient.

### BACKGROUND

Immunoglobulins (also known as antibodies) are specialized antigen binding proteins that are found in blood or other bodily fluids of vertebrates, and act primarily as a defense against invasion by foreign substances, such as bacteria and viruses. Antibodies can come in different varieties known as isotypes. In mammals there are five antibody isotypes known as IgA, IgD, IgE, IgG and IgM, each of which comprises different effector functions. IgG provides the majority of antibody-based immunity against invading pathogens.

Numerous disease and/or disorders are associated with immunodeficiency (*e.g.,* a reduction in IgG) or a defect in immune modulation (*e.g*. chronic inflammation). Treatments may include administration of agents that promote antibody proliferation and/or maturation. Additionally or alternatively, such treatments may also include administration of immunoglobulins to an immuno-compromised subject. However, methods for obtaining immunoglobulins from a donor require several processing steps at one or more facilities that are often at a different location from the donor and recipient delaying transfusion and leading to increased costs. As such, there exists a need for methods that allow immunoglobulins to be obtained from a donor and subsequently infused to a recipient without the need for off-site processing.

US-5,782,792 discloses a method for treating rheumatoid arthritis by removal of IgG and immune complexes from the patient's blood using an immunoadsorbent.

US-4,705,628 discloses an immunoglobulin adsorbent and an adsorption apparatus comprising the adsorbent.

WO 02/094344 discloses a method for removing an insulin binding protein from a biological fluid which comprises contacting the biological fluid with an affinity agent that is capable of forming a complex with the insulin binding protein.

### SUMMARY

According to the present invention there is provided a system for obtaining a pharmaceutically acceptable immunoglobulin product from blood of a donor according to claim 1.

Methods for obtaining immunoglobulins from blood are also described.

Immunoglobulins obtained from blood by the systems of the present disclosure may be used to treat numerous diseases or disorders associated with an immunodeficiency or a defect in immune modulation (*e.g*., Alzheimer's disease).

Systems are provided for obtaining a pharmaceutically acceptable immunoglobulin from blood of a donor comprising a first conduit configured to convey blood from the donor to a substrate, wherein said blood includes at least one first component and at least one second component, said first component of the blood including immunoglobulin, and wherein said substrate is adapted to bind immunoglobulin; and a second conduit configured to convey at least a portion of the second component of the blood from the first conduit to the donor.

In some examples, the immunoglobulin is IgG.

In some embodiments, the first conduit is configured to convey a component of the blood to the substrate. In some embodiments, the component is substantially plasma.

In some embodiments, the second component is substantially cellular. In some embodiments, the second component includes a second amount of the immunoglobulin. In some embodiments, the second amount of the immunoglobulin is less than the first amount of the immunoglobulin. In some embodiments, the second amount of the immunoglobulin is substantially zero. In some embodiments, the portion of the second component of the blood conveyed to the donor includes red blood cells. In some embodiments, the portion of the second component of the blood conveyed to the donor includes platelets.

The system comprises a third conduit configured to convey a product including a high concentration of immunoglobulin isolated from said substrate.

In some embodiments, the product (*e.g*., immunoglobulin) includes a therapeutically effective amount of immunoglobulin. In some embodiments, the third conduit is located in a facility different from a facility of the first conduit or the second conduit.

In some embodiments, the substrate with the bound immunoglobulin is capable of transfer to the facility of the third conduit.

A fourth conduit is also provided and configured to convey the product (*e.g.*, immunoglobulin) to a recipient.

In some embodiments, the product (*e.g*., immunoglobulin) is administered to a recipient without further processing. In some embodiments, the product is processed for administration to a recipient by at least one process. In some embodiments, the process is selected from the group consisting of adjusting the concentration of the immunoglobulin in the product, isolating at least a portion of IgG from the immunoglobulin, combining the product from said donor with a product from at least one other donor, reconstituting the immunoglobulin of the product in a liquid, sterilizing the product, and producing a pharmaceutically acceptable product.

In some embodiments, the product (*e.g.*, immunoglobulin) is further processed in a facility other than a facility of the first conduit or the second conduit.

In some embodiments, the product (*e.g*., immunoglobulin) is pooled with products obtained from less than ten donors using said system. In some embodiments, the product is pooled with products obtained from less than five donors. In some embodiments, immunoglobulin may be obtained and pooled from 1 to 10 or more donors.

Methods are also described for increasing the amount of protein obtained from a single donor for a protein product (*e.g*., immunoglobulin) comprising: removing blood from the donor, the blood including at least one first component and at least one second component, wherein said first component includes a protein; exposing the first component of the removed blood to a substrate adapted to bind said protein; returning at least a portion of the second component of the removed blood to the donor; and isolating from said substrate a product including a high concentration of said protein.

In some embodiments, the protein is an immunoglobulin. In further examples, the immunoglobulin is IgG.

In some embodiments, removing the blood from the donor includes removing an amount of blood from the donor sufficient to derive at least about 650 milliliters of plasma from the blood. In some embodiments, removing the blood from the donor includes removing at least two liters of blood from the donor. In some embodiments, removing the blood from the donor includes continuously removing blood from the donor until substantially an entire donor blood volume is exposed to the substrate at least once. In some embodiments, removing the blood from the donor includes continuously removing blood from the donor until an effective amount of the protein is bound to said substrate. In some embodiments, removing the blood from the donor includes sequentially removing at least two portions of the blood from the donor.

In some embodiments, at least one of the portions of the blood removed from the donor is at least about 500 milliliters.

In some embodiments, the first component includes the second component. In some embodiments, the first component is substantially plasma. In some embodiments, the second component is substantially cellular. In some embodiments, exposing the first component of the removed blood to the substrate includes exposing the first component of substantially all of the blood of the donor to the substrate at least once.

In some embodiments, the product includes a therapeutically effective amount of protein.

In some embodiments, the second component includes a second amount of the protein. In some examples, the second amount of the protein is less than the first amount of the protein. In other embodiments, the second amount of the protein is substantially zero.

In some embodiments, the methods include returning at least a portion of the first component of the removed blood to the donor. In some embodiments, the methods include treating the portion of the first component of the removed blood prior to returning said portion to the donor. In some embodiments, the methods include returning a portion of the removed blood not bound to the substrate to the donor. In some embodiments, the methods include returning substantially all of the second component of the removed blood to the donor. In some embodiments, the portion of the second component of the removed blood returned to the donor includes red blood cells. In some embodiments, the portion of the second component of the removed blood returned to the donor includes platelets. In some embodiments, at least the steps of removing the blood and exposing the first component of said blood to the substrate are repeated until an effective amount of the protein is bound to the substrate.

In some embodiments, an effective amount of the protein is capable of being isolated from the substrate.

In some embodiments, each of the steps of said method occurs in a same facility. In some embodiments, isolating the product from the substrate occurs in a facility other than at least one of the steps of said method.

In some embodiments, at least the steps of removing the blood and exposing the first component of said blood to the substrate occur in a fluid circuit adapted to operably connect the donor to the substrate.

In some embodiments, the methods include administering the product to a patient. In some embodiments, the product is obtained from each of less than ten donors. In some embodiments, the product is obtained from each of less than five donors.

In some embodiments, the product is administered to a patient without further processing. In some embodiments, the methods include preparing the product to be administered to a patient. In some embodiments, preparing the product includes at least one of the steps selected from the group consisting of adjusting the concentration of the protein in the product, isolating at least a portion of immunoglobulin from the protein, combining the product from said donor with a product from at least one other donor, reconstituting the protein of the product in a liquid, sterilizing the product, and producing a pharmaceutically acceptable product.

In some embodiments, preparing the product comprises at least one of adjusting the concentration of the protein in the product, isolating at least a portion of immunoglobulin from the protein, combining the product from said donor with a product from at least one other donor, reconstituting the protein of the product in a liquid, sterilizing the product, or producing a pharmaceutically acceptable product. In some embodiments, preparing the product to be administered to a patient occurs in a facility other than at least one of the other steps of said method.

Compositions are also described that comprise a protein product obtained from each of less than ten donors by the methods of the present disclosure.

In some embodiments, the protein product is obtained from each of less than five donors. In some embodiments, the composition is administered to a patient. In some embodiments, the composition is administered to a patient without further processing.

Methods are also described for treating Alzheimer's Disease comprising administering an effective amount of a composition, wherein said composition comprises a product obtained from at least one donor by the methods of the present disclosure.

In some embodiments, the composition comprises a product obtained from each of less than ten donors

Methods are described for increasing the amount of protein obtained from a single donor for a protein product comprising: removing blood from the donor in a first facility, the blood including at least one first component and at least one second component, wherein said first component includes a protein; exposing the first component of the removed blood to a substrate adapted to bind said protein in the first facility; returning at least a portion of the second component of the removed blood to the donor in the first facility; and isolating from said substrate a product including a high concentration of said protein in a second facility.

Methods are also described for treating a subject with a disease or disorder with one or more blood components by removing blood from a donor; separating one or more blood components from the blood of the donor on a substrate into a first fraction comprising one or more blood components for administration to the subject and a second fraction comprising one or more blood components are returned to the donor; obtaining the fraction of one or more blood components for administration to the subject; returning the second fraction comprising one or more blood components to the donor; pooling the first fraction comprising one or more blood components from one or more donors; and administering directly to the subject an effective amount of the pooled one or more blood components. In some embodiments, the first and the second fraction may be separated from whole blood. In other embodiments, the first and the second fraction may be separated from plasma. In some embodiments, the separation of the first and the second fraction is performed on a molecular level as opposed to a cellular level.

In some embodiments, the protein is an immunoglobulin. In further embodiments, the immunoglobulin is IgG.

In some embodiments, removing the blood from the donor includes removing an amount of blood from the donor sufficient to derive at least about 650 milliliters of plasma from the blood. In some embodiments, removing the blood from the donor includes removing at least two liters of blood from the donor. In some embodiments, removing the blood from the donor includes continuously removing blood from the donor until substantially an entire donor blood volume is exposed to the substrate at least once. In some embodiments, removing the blood from the donor includes continuously removing blood from the donor until an effective amount of the protein is bound to said substrate. In some embodiments, removing the blood from the donor includes sequentially removing at least two portions of the blood from the donor. In some embodiments, at least one of the portions of the blood removed from the donor is at least about 500 milliliters. In some embodiments, the first component includes the second component.

In some embodiments, the first component is substantially plasma. In some embodiments, the second component is substantially cellular.

In some embodiments, exposing the first component of the removed blood to the substrate includes exposing the first component of substantially all of the blood of the donor to the substrate at least once.

In some embodiments, the product includes a therapeutically effective amount of protein.

in some embodiments, the second component includes a second amount of the protein. In some embodiments, the second amount of the protein is less than the first amount of the protein. In some embodiments, the second amount of the protein is substantially zero.

In some embodiments, the methods include returning at least a portion of the first component of the removed blood to the donor. In some embodiments, the methods include treating the portion of the first component of the removed blood prior to returning said portion to the donor. In some embodiments, the methods include returning a portion of the removed blood not bound to the substrate to the donor. In some embodiments, the methods include returning substantially all of the second component of the removed blood to the donor. In some embodiments, the portion of the second component of the removed blood returned to the donor includes red blood cells. In some embodiments, the portion of the second component of the removed blood returned to the donor includes platelets. In some embodiments, at least the steps of removing the blood and exposing the first component of said blood to the substrate are repeated until an effective amount of the protein is bound to the substrate.

In some embodiments, an effective amount of the protein is capable of being isolated from the substrate.

In some embodiments, at least the steps of removing the blood and exposing the first component of said blood to the substrate occur in a fluid circuit adapted to operably connect the donor to the substrate.

In some embodiments, the methods include administering the product to a patient. In other examples, the product is obtained from each of less than ten donors. In some embodiments, the protein product is obtained from each of less than five donors. In still other embodiments, the product is administered to a patient without further processing. In some examples, the protein product is obtained from each of less than ten donors. In some embodiments, the protein product is obtained from each of less than five donors.

In some embodiments, the methods include preparing the product to be administered to a patient.

In some embodiments, preparing the product includes at least one of the steps selected from the group consisting of adjusting the concentration of the protein in the product, isolating at least a portion of immunoglobulin from the protein, combining the product from said donor with a product from at least one other donor, reconstituting the protein of the product in a solution, sterilizing the product, and producing a pharmaceutically acceptable product.

In some embodiments, preparing the product comprises at least one of adjusting the concentration of the protein in the product, isolating at least a portion of immunoglobulin from the protein, combining the product from said donor with a product from at least one other donor, reconstituting the protein of the product in a solution, sterilizing the product, or producing a pharmaceutically acceptable product.

In some embodiments, preparing the product to be administered to a patient occurs in a facility other than the first facility and the second facility.

Compositions are also described that comprise a protein product obtained from each of less than ten donors by the methods of the disclosure.

In some examples, the protein product is obtained from each of less than five donors. In some embodiments, the composition is administered to a patient. In still other embodiments, the composition is administered to a patient without further processing.

Uses of an immunoglobulin in the manufacture of a medicament obtained by the methods of the disclosure for the treatment of Alzheimer's disease are provided by the present disclosure.

### BRIEF DESCRIPTION OF THE DRAWINGS

Figure 1 shows the separation of plasma components from whole blood from a donor and the infusion of immunoglobulins from the plasma to a recipient.
Figure 2 shows the conveyance of blood components from a donor to a recipient.
Figure 3 shows the separation of immunoglobulins from whole blood from a donor and the infusion of the separated immunoglobulins to a recipient.
Figure 4 shows the conveyance of blood components from a donor to a recipient.

### DETAILED DESCRIPTION

The present disclosure provides systems for obtaining pharmaceutically acceptable immunoglobulins from blood of a donor for direct infusion to a recipient. It has been discovered that a substrate may be used to obtain, including isolate, immunoglobulins from a donor that may be used to directly infuse a recipient without the need for processing at one or more locations separate from the location of the donor and recipient. The isolated immunoglobulins may be used to treat, including ameliorate, Alzheimer's disease and/or diseases associated with an immunodeficiency and/or a defect in immune modulation.

The present disclosure provides systems for obtaining pharmaceutically acceptable immunoglobulin from blood of a donor which, in some examples, can then be directly infused to a recipient. The system comprises a first conduit configured to convey blood from the donor to a substrate, wherein said blood includes at least one first component and at least one second component, said first component of the blood including immunoglobulin, and wherein said substrate is adapted to bind immunoglobulin; and a second conduit configured to convey at least a portion of the second component of the blood from the first conduit to the donor. The first conduit may be configured to convey the first component of the blood to the substrate. The system includes a third conduit configured to convey a product including a high concentration of immunoglobulin isolated from said substrate. This third conduit may be located in a facility different from a facility of the first conduit or the second conduit. The system also includes a fourth conduit configured to convey the product to a recipient.

The present disclosure also provides methods for increasing the amount of protein obtained from a single donor for a protein product comprising: removing blood from the donor, the blood including at least one first component and at least one second component, wherein said first component includes a protein; exposing the first component of the removed blood to a substrate adapted to bind said protein; returning at least a portion of the second component of the removed blood to the donor; and isolating from said substrate a product including a high concentration of said protein. Optionally, the methods may include returning at least a portion of the first component of the removed blood to the donor. The methods may include administering the product (*e.g*., immunoglobulin) to a patient with or without further processing.

The present disclosure also provides methods for treating Alzheimer's disease and/or diseases or disorders associated with an immunodeficiency by administering an effective amount of a composition, wherein said composition comprises a product (*e.g.*, immunoglobulin) obtained from at least one donor by the methods of the present disclosure. The composition may be administered to a patient with or without further processing.

The present disclosure also provides methods for increasing the amount of protein (*e.g.*, immunoglobulin) obtained from a single donor for a protein product by: removing blood from the donor in a first facility, the blood including at least one first component and at least one second component, wherein said first component includes a protein; exposing the first component of the removed blood to a substrate adapted to bind said protein in the first facility; returning at least a portion of the second component of the removed blood to the donor in the first facility; and isolating from said substrate a product including a high concentration of said protein in a second facility.

The present disclosure also provides methods for treating a subject with a disease or disorder with one or more blood components by removing whole blood from a donor; separating one or more blood components from the whole blood of the donor on a substrate into a first fraction comprising one or more blood components (*e.g*., IgG) for administration to the subject and a second fraction comprising one or more blood components are returned to the donor; obtaining the fraction of one or more blood components for administration to the subject; returning the second fraction comprising one or more blood components to the donor; pooling the first fraction comprising one or more blood components from one or more donors; and administering directly to the subject an effective amount of the pooled one or more blood components. In some embodiments, the first and/or second fraction is a separation of proteins and/or molecules.

Although any methods and materials similar or equivalent to those described herein can be used in the practice or testing of the present disclosure, the preferred methods and materials are described. As used herein the singular forms "a", "and", and "the" include plural referents unless the context clearly dictates otherwise. All technical and scientific terms used herein have the same meaning as commonly understood to one of ordinary skill in the art to which this disclosure belongs unless clearly indicated otherwise.

### System for Obtaining Immunoglobulin from Blood

The present disclosure provides systems for, among other things, obtaining a pharmaceutically acceptable immunoglobulin (*e.g*., IgG) from blood from a donor for infusion into one or more recipients.

Systems may comprise a first conduit configured to convey blood from a donor to a substrate. The blood may include at least one first component (*e.g*., substantially plasma) and at least one second component (e.g., substantially cellular), wherein said first component of the blood includes immunoglobulin.

The substrate may be adapted to bind immunoglobulin (*e.g*., protein A). A first conduit may be configured to convey the first component (*e.g*., plasma) of the blood to the substrate. The first component of the removed blood may be exposed to the substrate until substantially all of the blood of the donor has been contacted with the substrate at least once. A second conduit may be configured to convey at least a portion of the second component of the blood from the first conduit to the donor. The steps of removing the blood and exposing the first component of said blood to the substrate may occur in a fluid circuit adapted to operably connect the donor to the substrate. A third conduit is configured to convey a product (*e.g*., immunoglobulin) isolated from said substrate. The third conduit may be located in a facility different from a facility of the first conduit or the second conduit. The substrate with the bound immunoglobulin may be capable of transfer to the facility of the third conduit. A fourth conduit is configured to convey the product to a recipient.

The systems of the present disclosure may be used to obtain, including isolate, immunoglobulin from a donor for infusion into a recipient (*e.g*., patient). An exemplary method may include: obtaining blood from a donor, processing the blood, isolating immunoglobulin from the blood, and infusing the immunoglobulin into a recipient or packaging the immunoglobulin for manufacture. Optionally, blood devoid of immunoglobulin may be transferred back into the donor. The method steps are discussed in more detail below.

### 1: Obtaining Blood from a Donor

Blood components (*e.g*., immunoglobulin, such as IgG) may be obtained from a donor by any known method in the art.

Potential donors may screened for anything that might make their blood unsafe to use. The screening may include testing for diseases that can be transmitted by a blood transfusion, including HIV and viral hepatitis. The donor may also asked about medical history and given a short physical examination to make sure that the donation is not hazardous to their health.

The amount of blood drawn and the methods vary, but a typical donation may be about 500 milliliters of whole blood. The collection can be done manually or with automated equipment that only takes specific portions of the blood. The blood may drawn from a vein and/or an artery. The blood may be mixed with sodium citrate, phosphate, dextrose, and/or adenine to keep the blood from clotting.

Immunoglobulin may be removed from a donor by continuously removing blood from the donor until an effective amount of immunoglobulin is bound to a substrate (see, *e.g*., PA column-based immunoadsorption (Immunosorba^{®}, Fresenius Medical Care, Germany) or antibody-based IgG immunoadsorption (Ig-Therasorb, PlasmaSelect, Teterow, Germany)). Suitable substrates may include Staphylococcus aureus Protein A or Streptococcus Protein G. A sufficient amount of blood may be removed from a donor to derive at least about 650 milliliters of plasma from the blood (*e.g*. at least two liters of blood). Blood may be removed from a donor until substantially an entire donor blood volume is exposed to the substrate at least once.

Columns have been coupled to Staphylococcus aureus Protein A, which binds to certain subclasses of human IgG (Immunosorba®, Excorim®, Lund, Sweden). In some examples, Protein A or Protein G may be coupled to Sepharose. The removal of certain subclasses of IgG is currently accomplished by perfusing the recipient's plasma over these S. aureus Protein A-coupled columns. The column matrix material may be sterilized by (1) a series of rinses with sterile pyrogen-free water to reduce bioburden, followed by (2) steam sterilization under conditions which will not melt the matrix material, preferably 115° C. for at least 20 minutes at <2 bar (until F₀ =6). All sterilization procedures may be carried out inside a sterilized isolator with glove boxes, placed inside a class 100,000 clean room.

The steps of removing blood and exposing a first component of the blood to the substrate are repeated until an effective amount of immunoglobulin, including, for example IgG, is bound to the substrate. An effective amount of immunoglobulin is that amount of immunoglobulin capable of treating a disease or disorder in a subject (e.g., a recipient).

### 2. Processing and Preparing Isolated Blood Components

Blood components (*e.g*., immunoglobulin), referred to herein as product, may be administered to a recipient with or without processing.

Processing may include, for example, adjusting the concentration of the immunoglobulin in the product, isolating at least a portion of IgG from the immunoglobulin, combining the product from said donor with a product from at least one other donor, reconstituting the immunoglobulin of the product in a liquid, sterilizing the product, and/or producing a pharmaceutically acceptable product.

Processing one or more blood components, such as immunoglobulin, may take place in a facility other than a facility of the first conduit or the second conduit.

The amount of protein (*e.g*., immunoglobulin, such as IgG) obtained from a single donor for a protein product may be increased by removing blood from the donor, the blood including at least one first component and at least one second component, wherein said first component includes a protein; exposing the first component of the removed blood to a substrate adapted to bind said protein; returning at least a portion of the second component of the removed blood to the donor; and isolating from said substrate a product including a high concentration of said protein.

The product may be prepared by adjusting the concentration of the protein in the product, isolating at least a portion of immunoglobulin from the protein, combining the product from said donor with a product from at least one other donor, reconstituting the protein of the product in a liquid, sterilizing the product, and/or producing a pharmaceutically acceptable product. Preparing the product to be administered to a patient may occur in a facility other than at least one of the other steps of said method.

### 3. Isolating Immunoglobulin from Blood

Immunoglobulin may be isolated from whole blood by using any method known in the art, including for example using immunoglobulin binding proteins such as Protein A. For example, suitable methods of using Protein A to bind IgG are described in U.S. Patent No. 5,817,528. When immobilized, Protein A can be used to purify antibody from serum or other samples or during immunoprecipitation to specifically bind an antibody/antigen complex in solution. The immunoglobulin binding protein may be affixed to a bead such as agarose. Bound immunoglobulin may be eluted by with low pH buffer.

Preferred elution buffers include:
(1) 5 mM mono-sodium citrate/10 mM citric acid, pH 2.8;
(2) 5 mM mono-sodium citrate/63 mM citric acid, pH 2.2;
(3) 5 mM sodium-acetate/acetic acid, pH 2.8;
(4) 0.1 m glycine-HCl pH 2.0 - 4.5;
(5) 0.5 M arginine pH 3.8 - 4.4 ; and
(6) 0.36 M arginine pH 4.4.

### 4. Infusion of Immunoglobulin to a Recipient

Immunoglobulin may be administered to a recipient, including, for example a human patient, in accordance with known methods, such as intravenous administration, *e.g.,* as a bolus or by continuous infusion over a period of time, by intramuscular, intraperitoneal, intracerebrospinal, subcutaneous, intra-articular, intrasynovial, intrathecal or oral routes. Intravenous, intraperitoneal, or subcutaneous administration of the immunoglobulin is preferred, with subcutaneous or intraperitoneal routes being particular preferred.

Blood devoid of one or more blood components (*e.g*., immunoglobulin) may be returned to a donor. A portion of the removed blood not bound to the substrate may be returned to the donor. The portion of the first component of the removed blood may be treated prior to returning the portion to the donor. Substantially all of the second component of the removed blood may be returned to the donor. The portion of the second component of the removed blood returned to the donor may include red blood cells and/or platelets.

### Treatment Methods

The present disclosure provides methods for treating diseases and/or disorders associated with an immunodeficiency by infusing one or more blood components obtained by the methods of the present disclosure into a recipient (*e.g*., patient).

Diseases that may be treated by the methods of the present disclosure include both primary and secondary immunodeficiency diseases. Exemplary secondary immunodeficiency diseases include, for example, lupus/SLE, fibromyalgia, autoimmune disease, diabetes, rheumatoid arthritis, multiple sclerosis, Chrohn's disease, AIDS, cancer, ITP, anemia, sarcoidosis, leukemia, EBV, HPV, and Reynauds.

Other diseases that may be treated by the methods of the present disclosure include Kowasocki syndrome, lupus, and Alzheimer's disease.

Other therapeutic regimens may be combined with the administration of the humanized vWF antibody. A combined administration includes co-administration, using separate formulations or a single pharmaceutical formulation, and consecutive administration in either order, wherein preferably there may be a time period while both (or all) active agents simultaneously exert their biological activities.

Selection of the preferred effective dose of immunoglobulin can be determined (e.g., via clinical trials) by a skilled artisan based upon the consideration of several factors which will be known to one of ordinary skill in the art. Such factors include the disease to be treated or prevented, the symptoms involved, the patient's body mass, the patient's immune status and other factors known by the skilled artisan to reflect the accuracy of administered pharmaceutical compositions.

The precise dose to be employed in the formulation will also depend on the route of administration, and the seriousness of the cancer, and should be decided according to the judgment of the practitioner and each patient's circumstances. Effective doses may be extrapolated from dose-response curves derived from *in vitro* or animal model test systems.

For immunoglobulin, the dosage administered to a patient is typically 0.1 mg/kg to 100 mg/kg of the patient's body weight. Preferably, the dosage administered to a patient is between 0.1 mg/kg and 20 mg/kg of the patient's body weight, more preferably 1 mg/kg to 10 mg/kg of the patient's body weight. Generally, human and humanized immunoglobulin have a longer half-life within the human body than antibodies from other species due to the immune response to the foreign polypeptides. Thus, lower dosages of human immunoglobulin and less frequent administration is often possible.

Therapeutically effective amount or effective amount can refer to an amount effective to ameliorate or prevent the symptoms, or prolong the survival of the subject being treated. Determination of a therapeutically effective amount is well within the capabilities of those skilled in the art, especially in light of the detailed disclosure provided herein. A therapeutically effective amount as described herein includes an amount of immunoglobulin effective to treat a disease or disorder associated with immunodeficiency in a subject.

Therapeutically effective dose or effective dose can refer to a dose effective to ameliorate or prevent the symptoms, or prolong the survival of a subject being treated. A therapeutically effective dose as described herein includes a dose of immunoglobulin effective to treat a disease or disorder associated with immunodeficiency in a subject.

### Pharmaceutical Compositions

Pharmaceutical formulations comprising one or more isolated blood components (*e.g*., immunoglobulin, such as IgG) are provided. Formulations of immunoglobulin may be prepared for storage by mixing an immunoglobulin having the desired degree of purity with optional pharmaceutically acceptable carriers, excipients, or stabilizers (Remington's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low-molecular-weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g*. Zn-protein complexes); and/or non-ionic surfactants such as TWEEN™, PLURONICS™ or polyethylene glycol (PEG).

The formulation herein may also contain more than one active compound as necessary for the particular indication being treated, preferably those with complementary activities that do not adversely affect each other.

The immunoglobulin may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug-delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semipermeable matrices of solid hydrophobic polymers containing the immunoglobulin, which matrices are in the form of shaped articles, *e.g.* films, or microcapsules. Examples of sustained-release matrices include polyesters, hydrogels (*e.g*., poly(2-hydroxyethyl-methacrylate), or poly(vinylalcohol)), polylactides (U.S. Pat. No. 3,773,919), copolymers of L-glutamic acid and y ethyl-L-glutamate, nondegradable ethylene-vinyl acetate, degradable lactic acid-glycolic acid copolymers such as the LUPRON DEPO™ (injectable microspheres composed of lactic acid-glycolic acid copolymer and leuprolide acetate), and poly-D-(-)-3-hydroxybutyric acid.

The formulations to be used for *in vivo* administration should be sterile. This may be accomplished by filtration through sterile filtration membranes.

### Articles of Manufacture

Articles of manufacture containing one or more blood components are provided. The article of manufacture may comprise a container and a label or package insert on or associated with the container. Suitable containers include, for example, bottles, vials or syringes. The containers may be formed from a variety of materials such as glass or plastic. The container holds a composition that may be effective for treating a disease or disorder associated with immunodeficiency and may have a sterile access port (e.g., the container may be an intravenous solution bag or a vial having a stopper pierceable by a hypodermic injection needle). At least one active agent in the composition may be the isolated immunoglobulin described herein. The label or package insert may indicate that the composition may be used for treating the condition of choice, such as immunodeficiency. In one embodiment, the label or package insert may indicate that the composition comprising the immunoglobulin may be used to treat a disease or disorder associated with immunodeficiency.

Moreover, the article of manufacture may comprise (a) a first container with a composition contained therein, wherein the composition comprises the immunoglobulin herein, and (b) a second container with a composition contained therein, wherein the composition comprises a therapeutic agent other than the immunoglobulin. The article of manufacture in this embodiment of the disclosure may further comprise a package insert indicating that the first and second compositions can be used in combination to treat a disease or disorder associated with immunodeficiency. Alternatively, or additionally, the article of manufacture may further comprise a second (or third) container comprising a pharmaceutically acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

### EXAMPLES

### Example 1: Infusion of Immunoglobulin from Plasma in Donor to a Recipient

Immunoglobulin may be obtained, including isolated, from a donor and be used to infuse a recipient.

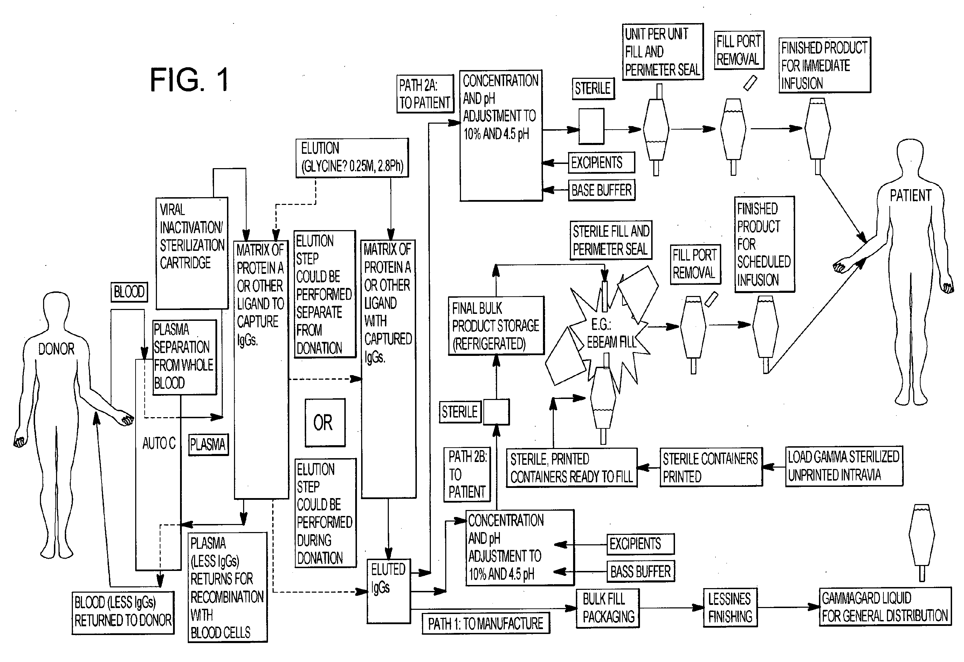

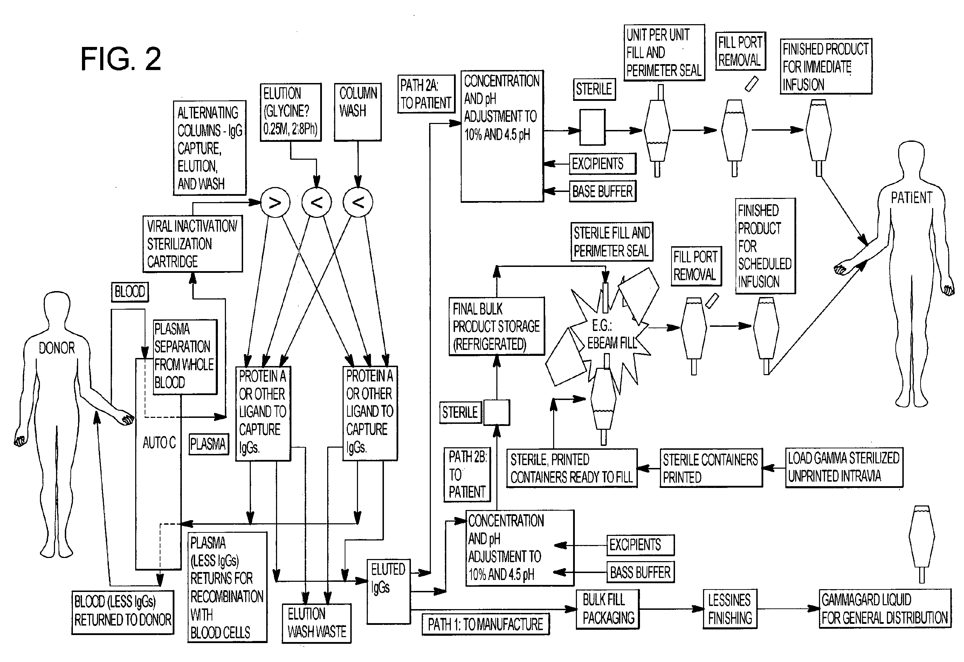

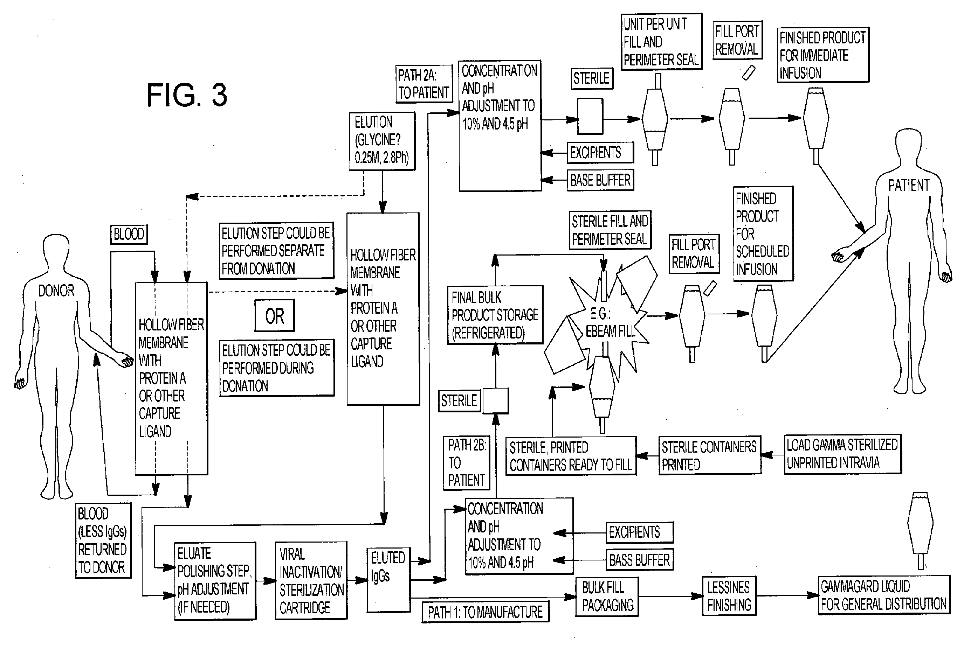

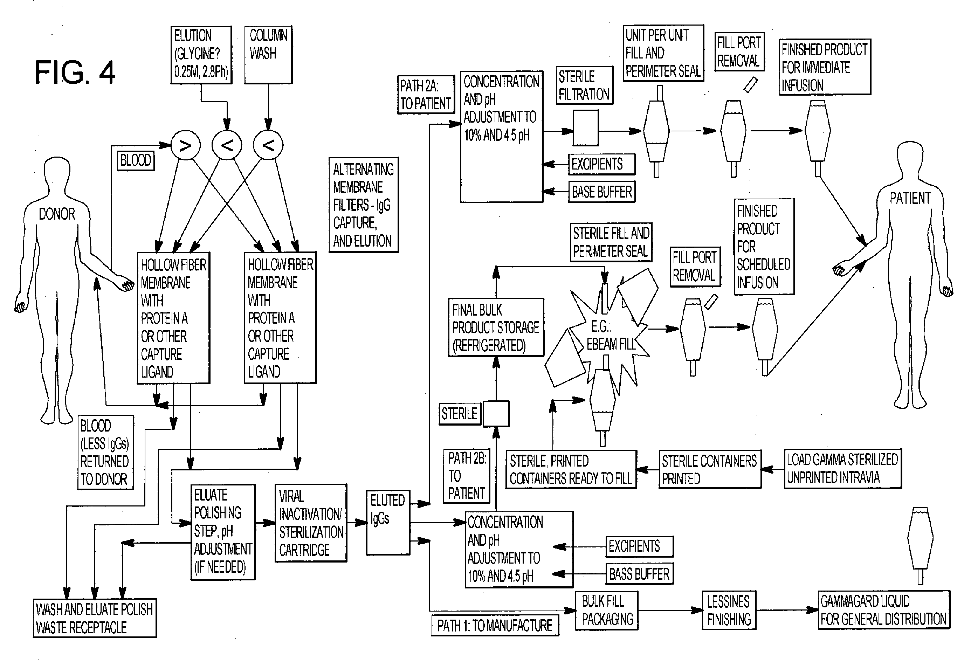

In an exemplary method, blood is first obtained from a recipient by any known method in the art. Plasma is then separated from the donor's blood and sterilized to inactivate virus. Next, the sterilized plasma is contacted with a matrix of protein A. Alternatively, alternating columns may be used to capture IgG. The columns may be washed with the eluted IgGs entering another column. (see, for example Figure 2). Alternatively, a hollow fiber membrane with Protein A or another capture ligand for immunoglobulin may be used (see, e.g., Figures 3 and 4). Optionally, plasma less IgG and blood may be returned to the donor. Captured IgG may be eluted from the matrix during donation of blood or separate from donation. Elution may be preformed separate from donation or at the same time as donation. The eluted IgGs may be either used to infuse a recipient or used in the manufacture of IgG for bulk distribution. Eluted IgGs may be concentrated to 10% and pH adjusted to a slightly acidic pH, for example pH 4.5. Next, excipients and buffers may be added to the IgG to a product a pharmaceutical composition. The compositions may be placed into sterile containers and used to infuse a recipient. (See, for example, Figure 1).

Aspects of the present subject matter described above may be beneficial alone or in combination, with one or more other aspects. Without limiting the foregoing description, in accordance with one aspect of the subject matter herein, there is provided a system for obtaining a pharmaceutically acceptable immunoglobulin from blood of a donor according to claim 1.

In accordance with another aspect which may be used or combined with any of the preceding aspects, the immunoglobulin is IgG.

In accordance with another aspect which may be used or combined with any of the preceding aspects, the first conduit is configured to convey the first component of the blood to the substrate.

In accordance with another aspect which may be used or combined with any of the preceding aspects, the first component is substantially plasma.

In accordance with another aspect which may be used or combined with any of the preceding aspects, the second component is substantially cellular.

In accordance with another aspect which may be used or combined with any of the preceding aspects, the second component includes a second amount of the immunoglobulin.

In accordance with another aspect which may be used or combined with the preceding aspect, the second amount of the immunoglobulin is less than the first amount of the immunoglobulin.

In accordance with another aspect which may be used or combined with the preceding aspect, the second amount of the immunoglobulin is substantially zero.

In accordance with another aspect which may be used or combined with any of the preceding aspects, the portion of the second component of the blood conveyed to the donor includes red blood cells.

In accordance with another aspect which may be used or combined with the preceding aspect, the portion of the second component of the blood conveyed to the donor includes platelets.

In accordance with another aspect which may be used or combined with any of the preceding aspects, the product includes a therapeutically effective amount of immunoglobulins.

In accordance with another aspect which may be used or combined with any of the preceding aspects, the third conduit is located in a facility different from a facility of the first conduit or the second conduit.

In accordance with another aspect which may be used or combined with any of the preceding two aspects, the substrate with the bound immunoglobulin is capable of transfer to the facility of the third conduit.

In accordance with another aspect which may be used or combined with any of the precedin three aspects, the product is administered to a recipient without further processing.

In accordance with another aspect which may be used or combined with any of the preceding four aspects, the product is processed for administration to a recipient by at least one process.

In accordance with another aspect which may be used or combined with the preceding aspect, the process is selected from the group consisting of adjusting the concentration of the immunoglobulin in the product, isolating at least a portion of IgG from the immunoglobulin, combining the product from said donor with a product from at least one other donor, reconstituting the immunoglobulin of the product in a liquid, sterilizing the product, and producing a pharmaceutically acceptable product.

In accordance with another aspect which may be used or combined with any of the preceding two aspects, the product is further processed in a facility other than a facility of the first conduit or the second conduit.

In accordance with another aspect which may be used or combined with any of the preceding eight aspects, the product is pooled with products obtained from less than ten donors using said system or the product is pooled with products obtained from less than five donors using said system.

In accordance with another aspect which may be used or combined with any of the preceding aspects, the first fraction and the second fraction are separated from plasma.

In accordance with another aspect which may be used or combined with any of the preceding aspects, the separation of the first and the second fraction is performed on a molecular level.

While the present disclosure has been described and illustrated herein by references to various specific, materials, procedures and examples, it is understood that the disclosure is not restricted to the particular combinations of material and procedures selected for that purpose. Numerous variations of such details can be implied as will be appreciated by those skilled in the art. It is intended that the specification and examples be considered as exemplary, only.

## Claims

1. A system for obtaining a pharmaceutically acceptable immunoglobulin product from blood of a donor comprising:
a substrate that is adapted to bind immunoglobulin;
a first conduit configured to convey blood or a blood component from the donor to the substrate, wherein said blood or blood component includes at least one first component and at least one second component, said first component including immunoglobulin;
a second conduit configured to convey at least a portion of the second component of the blood or blood component from the first conduit to the donor:
a third conduit configured to convey an immunoglobulin product isolated from said substrate; and
a fourth conduit configured to convey the product directly to a recipient.

2. The system of claim 1, wherein the substrate binds IgG.

3. The system of claim 1, further comprising a separator for separating plasma containing immunoglobulin from whole blood.

## Patentansprüche

1. System zum Erhalten eines pharmazeutisch verträglichen Immunglobulin-Produkts aus Blut eines Spenders, umfassend:
ein Substrat, das adaptiert ist, um Immunglobulin zu binden;
ein erster Kanal, welcher konfiguriert ist, um Blut oder einen Blutbestandteil vom Spender zum Substrat zu leiten, wobei das Blut oder der Blutbestandteil mindestens einen ersten Bestandteil und mindestens einen zweiten Bestandteil einschließt, wobei der erste Bestandteil Immunglobulin einschließt;
ein zweiter Kanal, welcher konfiguriert ist, um mindestens einen Teil des zweiten Bestandteils des Bluts oder des Blutbestandteils von dem ersten Kanal zum Spender zu leiten;
ein dritter Kanal, welcher konfiguriert ist, um ein Immunglobulin-Produkt, welches aus dem Substrat isoliert wurde, zu leiten; und
ein vierter Kanal, welcher konfiguriert ist, um das Produkt direkt an den Empfänger zu leiten.

2. System nach Anspruch 1, wobei das Substrat IgG bindet.

3. System nach Anspruch 1, zudem umfassend eine Trennvorrichtung, um Immunglobulin enthaltendes Plasma von Vollblut zu trennen.

## Revendications

1. Système pour obtenir un produit d'immunoglobuline pharmaceutiquement acceptable à partir du sang d'un donneur, comprenant :
un substrat qui est adapté pour lier l'immunoglobuline ;
un premier conduit configuré pour convoyer le sang ou un composant sanguin du donneur au substrat, dans lequel ledit sang ou composant sanguin comprend au moins un premier composant et au moins un second composant, ledit premier composant comprenant de l'immunoglobuline ;
un second conduit configuré pour convoyer au moins une partie du second composant du sang ou composant sanguin du premier conduit au donneur ;
un troisième conduit configuré pour convoyer un produit d'immunoglobuline isolé dudit substrat ; et
un quatrième conduit configuré pour convoyer le produit directement à un destinataire.

2. Système selon la revendication 1, dans lequel le substrat se lie à l'IgG.

3. Système selon la revendication 1, comprenant en outre un séparateur pour séparer le plasma contenant de l'immunoglobuline du sang total.
